(19) Europäisches Patentamt
European Patent Office
Office européen des brevets

(11) Veröffentlichungsnummer: **0 171 502**
**A2**

(12) # EUROPÄISCHE PATENTANMELDUNG

(21) Anmeldenummer: 85103302.7

(22) Anmeldetag: 21.03.85

(51) Int. Cl.⁴: **C 07 D 249/08**
C 07 D 233/56, C 07 D 231/12
C 07 D 249/10, C 07 D 233/68
C 07 D 231/16, A 01 N 47/38

(30) Priorität: 28.03.84 DE 3411388

(43) Veröffentlichungstag der Anmeldung:
19.02.86 Patentblatt 86/8

(84) Benannte Vertragsstaaten:
AT BE CH DE FR GB IT LI NL SE

(71) Anmelder: BASF Aktiengesellschaft
Carl-Bosch-Strasse 38
D-6700 Ludwigshafen(DE)

(72) Erfinder: Rentzea, Costin, Dr. Dipl.-Ing.
Richard-Kuhn-Strasse 1-3
D-6900 Heidelberg(DE)

(72) Erfinder: Spiegler, Wolfgang, Dr.
Westpreussenstrasse 5
D-6520 Worms 27(DE)

(72) Erfinder: Himmele, Walter, Dr.
Eichenweg 14
D-6909 Walldorf(DE)

(72) Erfinder: Ammermann, Eberhard, Dr.
Sachsenstrasse 3
D-6700 Ludwigshafen(DE)

(72) Erfinder: Pommer, Ernst-Heinrich, Dr.
Berliner Platz 7
D-6703 Limburgerhof(DE)

(54) **Vinylazolylharnstoffe und diese enthaltende Fungizide.**

(57) Vinylharnstoffe der Formel

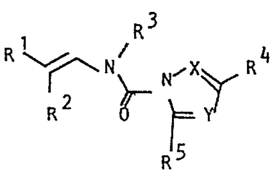

I,

in der $R^1$ und $R^2$ Wasserstoff oder einen gegebenenfalls substituierten Alkyl- oder Cycloalkylrest bedeuten oder $R^1$ zusammen mit $R^2$ Teil eines gegebenenfalls substituierten carbocyclischen Ringes ist, $R^3$ einen gegebenenfalls substituierten Alkyl- oder Cycloalkylrest,
X Stickstoff oder die Gruppe C–$R^6$,
Y Stickstoff oder CH,
$R^4$, $R^5$ und $R^6$ Wasserstoff, Halogen oder Alkyl bedeuten, und diese enthaltende Fungizide.

EP 0 171 502 A2

BEZEICHNUNG GEÄNDERT
siehe Titelseite

Vinylharnstoffe und diese enthaltende Fungizide

Die vorliegende Erfindung betrifft neue Vinyl-azolylharnstoffe, Verfahren zu ihrer Herstellung und fungizide Mittel, die diese Verbindungen als Wirkstoffe enthalten.

Es ist bekannt, N-Trichlormethylthio-tetrahydrophthalimid als Fungizid in der Landwirtschaft sowie im Obst- und Gartenbau einzusetzen (Chem. Week, June 21, 1972, Seite 46). Das bekannte Mittel ist jedoch nur vor der Infektion verwendbar und seine Wirkung genügt bei niedrigen Aufwandmengen den Anforderungen der Praxis nicht.

Es wurde nun gefunden, daß Vinyl-azolylharnstoffe der Formel

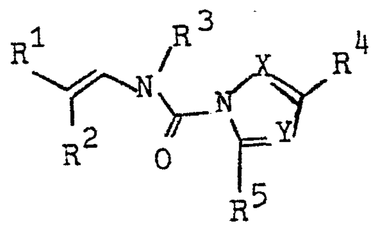

I,

in der $R^1$ und $R^2$ gleich oder verschieden sind und Wasserstoff oder einen gegebenenfalls durch Halogen oder Alkoxy substituierten Alkyl- oder Cycloalkylrest jeweils mit bis zu 12 Kohlenstoffatomen bedeuten oder $R^1$ zusammen mit $R^2$ Teil eines gegebenenfalls durch eine oder mehrere Alkylgruppen mit 1 bis 3 Kohlenstoffatomen substituierten 5- oder 6-gliedrigen carbocyclischen Ringes ist,
$R^3$ einen gegebenenfalls durch Halogen oder Alkoxy substituierten Alkyl- oder Cycloalkylrest jeweils mit bis zu 12 Kohlenstoffatomen,
X Stickstoff oder die Gruppe $C-R^6$
Y Stickstoff oder CH, wobei mindestens einer der beiden Reste X und Y Stickstoff bedeutet,
$R^4$, $R^5$ und $R^6$ gleich oder verschieden sind und Wasserstoff, Halogen oder Alkyl mit bis zu 5 Kohlenstoffatomen bedeuten, sehr gut wirksam gegen Schadpilze sind.

Die neuen Verbindungen enthalten Doppelbindungen und gegebenenfalls chirale Zentren in den Substituenten $R^1$, $R^2$ und/oder $R^3$ und werden im allgemeinen in Form von Z- und E-Isomerengemische und gegebenenfalls als Racemate oder als Diastereomerengemische erhalten. Die Z- und E-Isomeren lassen sich bei einigen der erfindungsgemäßen Verbindungen beispielsweise durch Säulenchromatographie oder aufgrund von Löslichkeitsunterschieden in reiner Form isolieren. Aus einheitlichen Diastereomeren kann

man ferner mit bekannten Methoden einheitliche Racemate und Enantiomere erhalten. Alle diese Isomeren und ihre Gemische werden von der vorliegenden Erfindung umfaßt. Für die Anwendung der erfindungsgemäßen Verbindungen als Fungizide sind sowohl die einheitlichen Diasteromeren, Enantiomeren bzw. geometrische Isomeren Z und E wie auch deren bei der Synthese anfallenden Gemische geeignet. Bevorzugt werden die letzteren verwendet.

$R^1$ und $R^2$ bedeuten beispielsweise Wasserstoff, Methyl Ethyl, Propyl, Isopropyl, 2-Chlorpropyl, 3-Methoxypropyl, 3-Butoxypropyl, Butyl, sek.-Butyl, iso-Butyl, tert.-Butyl, n-Pentyl, 2-Methylbutyl, 2,2-Dimethylpropyl, n-Hexyl, 2,2,3-Trimethylpropyl, 3,3-Dimethylbutyl, n-Heptyl, n-Octyl, 2,4,4-Trimethylpentyl, 2,2,3,3-Tetramethylbutyl, 2-Ethylhexyl, n-Nonyl, 3,5,5-Trimethylhexyl, 2-Isopropyl-5-methylhexyl, n-Decyl, 3,7-Dimethyloctyl, Dodecyl, Cyclopentyl, Cyclohexyl, 4-tert.-Butylcyclohexyl oder $R^1$ zusammen mit $R^2$ Teil eines gegebenenfalls durch Methyl, Ethyl, Propyl, Isopropyl, n-Butyl, tert.-Butyl, substituierten Cyclopentyl- oder Cyclohexylringes ist. Alkoxy ist beispielsweise $C_1$-$C_4$-Alkoxy.

Als Bedeutung für $R^3$ in Formel I kommen Reste wie tert.-Butyl, 2-Methylbutyl-2, 2-Ethylpentyl-2, 1,1,2-Trimethylpropyl-1, 1,1-Dimethylbutyl-1, 1,1-Dimethylpentyl-1, 1,1,2,2-Tetramethylpropyl-1, 1-Methyl-1cyclopentyl, 1-Ethyl-1-cyclopentyl, 1-Propyl-1-cyclopentyl, 1-Methyl-1-cyclohexyl, 1-Ethyl-1-cyclohexyl, 1,4-Dimethyl-1-cyclohexyl, 1-Methyl-4-tert.-butyl-1-cyclohexyl, 1-Ethyl-4-methyl-1-cyclohexyl oder 1,2,4,6-Tetramethyl-1-cyclohexyl in Frage.

Die Gruppen $R^4$, $R^5$ und $R^6$ bedeuten beispielsweise Wasserstoff, Chlor, Brom, Methyl, Ethyl, n-Propyl oder Isopropyl, ferner bedeutet X beispielsweise Stickstoff oder die Gruppe C-$R^6$ und Y bedeutet Stickstoff oder CH.

Man erhält die Vinylharnstoffe der Formel I beispielsweise durch Umsetzung von einer Verbindung der Formel

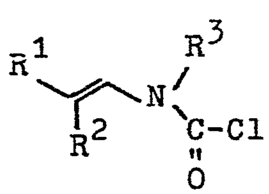

II,

in der $R^1$, $R^2$ und $R^3$ die oben angegebenen Bedeutungen haben,

a) mit Azolen der Formel

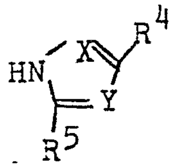 III,

in der $R^4$, $R^5$, X und Y die oben angegebenen Bedeutungen haben, oder

b) mit deren Metall-Derivaten der Formel

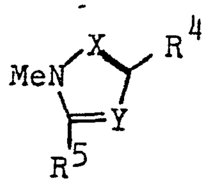 (IV),

in der $R^4$, $R^5$, X und Y die oben angegebenen Bedeutungen haben und Me Lithium, Natrium, Kalium oder 1/2-Calcium bedeutet, oder

c) mit deren Silyl-Derivaten der Formel

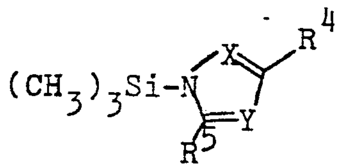 (V),

in der $R^4$, $R^5$, X und Y die oben angegebenen Bedeutungen haben.

Die Umsetzungen a) und b) werden bevorzugt.

Die Reaktion a) erfolgt gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120°C.

Als bevorzugte, gegenüber den Reaktionsteilnehmern inerte Lösungs- oder Verdünnungsmittel können beispielsweise aliphatische oder aromatische, gegebenenfalls halogenierte Kohlenwasserstoffe, wie n-Pentan, Cyclohexan, Methylenchlorid, 1,1,1-Trichlorethan, Benzol, Toluol, Xylol, Chlorbenzol, aliphatische Ketone, wie Aceton, Methylethylketon, Diethylketon, Ether, wie Diethylether, Methyl-tert.-butylether, Dimethoxyethan, Tetrahydrofuran oder Dioxan, Ester, wie Essigsäureethylester, Nitrile wie Acetonitril, Amide, wie Dimethylformamid, Dimethylacetamid und N-Methylpyrrolidon oder Gemische dieser Lösungsmittel verwendet werden.

Geeignete Basen, die gegebenenfalls auch als säurebindende Mittel bei der Reaktion verwendet werden können, sind beispielsweise Alkalihydroxide wie Lithium-, Natrium-, Kalium- oder Calciumhydroxid, Alkalicarbonate wie Natrium- oder Kaliumcarbonat oder Natrium- und Kaliumhydrogencarbonat, Amine wie Triethylamin, Tripropylamin, N-Methylpyrrolidin, N-Methylpiperidin, N,N-Dimethylcyclohexylamin, N,N'-Tetramethylethylendiamin, N,N-Dimethylanilin, N,N-Diethylanilin, Pyridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammonium-chlorid, -bromid oder -iodid, Benzyltriethylammonium-chlorid oder -bromid oder Kronenether wie 12-Krone-4, 15-Krone-5, 18-Krone-6 oder Dibenzo-18-krone-6 in Frage.

Die Reaktionen b) und c) erfolgen gegebenenfalls in Gegenwart eines Lösungs- oder Verdünnungsmittels bei einer Temperatur im Bereich zwischen 0 und 140°C, vorzugsweise zwischen 0 und 100°C. Hierfür eignen sich dieselben Lösungsmittel wie für die Verfahrensvariante a).

Die Ausgangsstoffe der Formel II können leicht nach bekannten Verfahren hergestellt werden, beispielsweise durch Umsetzung von aliphatischen bzw. cycloaliphatischen Schiff'schen Basen mit Phosgen (DE-OS 1.901.542; siehe auch J. P. Chupp, J. Heterocyl. Chem., 8, Seite 677 (1971)).

Für die Herstellung von Verbindungen der Formel I und von Ausgangsverbindungen der Formel IV und V können beispielsweise die folgenden Azole der Formel III eingesetzt werden: Imidazol, 2-Methyl-imidazol, 4(5)-Methylimidazol, 2,4-Dimethylimidazol, 2,4,5-Trimethylimidazol, 4,5-Dichlorimidazol, 4,5-Dibromimidazol, 2,4,5-Trichlorimidazol, 2,4,5-Tribromimidazol, 2-Ethylimidazol, 2-Propylimidazol, 2-Isopropylimidazol, 2,4-Diethylimidazol, Pyrazol, 3,5-Dimethylpyrazol, 1,2,4-Triazol, 3-Chlor-1,2,4-triazol, 3,5-Dichlor-1,2,4-triazol, 3,5-Dibrom-1,2,4-triazol, 3,5-Dimethyl-1,2,4-triazol und 1,2,3-Triazol.

Die folgenden Beispiele erläutern die Herstellung der Verbindungen der Formel I:

Beispiel 1

Zu einer Lösung von 18,1 g (0,22 Mol) 2-Methylimidazol in 150 ml trockenem Tetrahydrofuran werden bei 20°C 20,3 g (0,1 Mol) N-(2-Isopropylvinyl)-N-tert.-butylcarbamoylchlorid zugetropft. Nach 8stündigem Rühren

bei 70°C wird das Gemisch auf 20°C abgekühlt und der gebildete Niederschlag abgesaugt. Das Filtrat wird unter vermindertem Druck eingeengt, der Rückstand in 200 ml Methylenchlorid aufgenommen, dreimal mit je 80 ml Wasser gewaschen, getrocknet und eingeengt. Das zurückbleibende Öl wird in Vakuum destilliert.

Man erhält 17,2 g 1-(N-Isopropylvinyl)-N-tert.-butylcarbamoyl)-2-methylimidazol als farblose Flüssigkeit vom Sdp. 105 - 106°C/0,1 mbar und $n_D^{25}$ 1,4893 (Verbindung Nr. 1).

Beispiel 2

Zu einer Suspension von 10,5 g (0,115 Mol) Natrium-1,2,4-triazolid in 100 ml trockenem Tetrahydrofuran werden bei 20°C 21 g (0,103 Mol) N--(2-Isopropylvinyl)-N-tert.-butylcarbamoylchlorid zugetropft. Nach 6stündigem Rühren bei 65°C wird das Gemisch auf 20°C abgekühlt und der gebildete Niederschlag abgesaugt. Das Filtrat wird eingeengt und das zurückbleibende Öl im Vakuum destilliert. Man erhält 13,6 g 1-(N-(2-Isopropylvinyl)-N-tert.-butylcarbamoyl)-1,2,4-triazol als farblose Flüssigkeit vom Sdp. 95 - 97°C/0,15 mbar und $n_D^{25}$ 1,4815 (Verbindung Nr. 2).

Beispiel 3

Zu einer Suspension von 42 g Kaliumcarbonat in 150 ml trockenem Methylenchlorid werden bei 20°C nacheinander 27,2 g (0,2 Mol) Pyrazol, 1 g 4-Dimethylamminopyridin und 40,6 g (0,2 Mol) N-(2-Isopropylvinyl)-N--tert.-butylcarbamoylchlorid zugesetzt. Nach 10stündigem Rühren bei 40°C wird der gebildete Niederschlag abgesaugt und mit 200 ml Methylenchlorid gewaschen. Das Filtrat wird dreimal mit je 80 ml Wasser gewaschen, über $Na_2SO_4$ getrocknet und unter vermindertem Druck eingeengt. Das zurückbleibende Öl wird im Vakuum eingeengt. Man erhält 30,3 g 1-(N-(2-Isopropylvinyl)-N-tert.-butylcarbamoyl)-pyrazol als farblose Flüssigkeit vom Sdp. 104 - 105°C/0,3 mbar und $n_D^{20}$ 1,4828 (Verbindung Nr. 3).

In entsprechender Weise können die in der folgenden Tabelle aufgeführten Verbindungen hergestellt werden:

| Verb. Nr. | $R^1$ | $R^2$ | $R^3$ | $R^4$ | $R^5$ | X | Y | Phys. Konstante |
|---|---|---|---|---|---|---|---|---|
| 4 | $CH_3-$ | H | tert.-Butyl | H | H | CH | N | Sdp. 90 - 91°C/ 0,2 mbar |
| 5 | $C_2H_5-$ | H | tert.-Butyl | H | H | CH | N | Sdp. 90 - 93°C/ 0,15 mbar |
| 6 | $n-C_3H_7-$ | H | tert.-Butyl | H | H | CH | N | Sdp. 115 - 117°C/ 0,3 mbar |
| 7 | $n-C_4H_9-$ | H | tert.-Butyl | H | H | CH | N | Sdp. 129 - 131°C/ 0,4 mbar |
| 8 | $n-C_6H_{13}-$ | H | tert.-Butyl | H | H | CH | N | Sdp. 135 - 137°C/ 0,5 mbar |
| 9 | $n-C_6H_{13}$ | H | tert.-Butyl | H | H | N | N | Sdp. 130 - 131°C/ 0,5 mbar |
| 10 | $n-C_{10}H_{21}$ | H | tert.-Butyl | H | H | CH | N | Sdp. 144 - 146°C/ 0,4 mbar |
| 11 | $n-C_{10}H_{21}$ | H | tert.-Butyl | H | H | N | N | Sdp. 141 - 142°C/ 0,4 mbar |
| 12 | $(CH_3)_3CCH_2CH(CH_3)CH_2-$ | H | tert.-Butyl | H | H | CH | N | Sdp. 138 - 140°C/ 0,5 mbar |
| 13 | $(CH_3)_3CCH_2CH(CH_3)CH_2-$ | H | tert.-Butyl | H | H | N | N | Sdp. 134 - 136°C/ 0,5 mbar |

| Verb. Nr. | $R^1$ | $R^2$ | $R^3$ | $R^4$ | $R^5$ | X | Y | Phys. Konstante |
|---|---|---|---|---|---|---|---|---|
| 14 | $i-C_3H_7$ | H | tert.-Butyl | H | $C_2H_5-$ | CH | N | $n_D^{25}$ 1,4870 |
| 15 | $i-C_3H_7$ | H | tert.-Butyl | H | $i-C_3H_7-$ | CH | N | $n_D^{25}$ 1,4859 |
| 16 | $i-C_3H_7$ | H | tert.-Butyl | Cl | H | C-Cl | N | Sdp. 130 - 132°C/ 0,1 mbar |
| 17 | $i-C_3H_7$ | H | tert.-Butyl | $CH_3$ | $CH_3$ | $C-CH_3$ | N | Sdp. 110 - 113°C/ 0,1 mbar |
| 18 | $i-C_3H_7$ | H | tert.-Butyl | Cl | H | N | N | Fp. 73 - 75°C |
| 19 | $i-C_3H_7$ | H | tert.-Butyl | $CH_3$ | $CH_3$ | N | N | Sdp. 109 - 113°C/ 0,2 mbar |
| 20 | $i-C_3H_7$ | H | tert.-Butyl | $CH_3$ | $CH_3$ | N | CH | $n_D^{25}$ 1,4855 |
| 21 | $i-C_3H_7$ | H | $-C(CH_3)_2CH_2CH_3$ | H | H | CH | N | $n_D^{25}$ 1,4935 |
| 22 | $i-C_3H_7$ | H | $-C(H_3)_2CH_2CH_3$ | H | $CH_3$ | CH | N | $n_D^{25}$ 1,4924 |
| 23 | $i-C_3H_7$ | H | $-C(CH_3)_2CH_2CH_3$ | H | H | N | N | $n_D^{25}$ 1,4850 |
| 24 | $i-C_3H_7$ | H | $-C(CH_3)_2CH_2CH_3$ | H | H | N | CH | $n_D^{25}$ 1,4895 |
| 25 | $i-C_3H_7$ | H | $-C(C_2H_5)_3$ | H | H | CH | N | Fp. 54 - 56 °C |
| 26 | $i-C_3H_7$ | H | $-C(C_2H_5)_3$ | H | $CH_3$ | CH | N | Sdp. 150 - 152°C/ 0,4 mbar |
| 27 | $i-C_3H_7$ | H | $-C(C_2H_5)_3$ | H | H | N | N | Sdp. 134 - 136°C/ 0,4 mbar |
| 28 | $i-C_3H_7$ | H | $-C(C_2H_5)_3$ | H | H | N | CH | Sdp. 126 - 128°C/ 0,5 mbar |
| 29 | $i-C_3H_7$ | H | $-C(CH_3)_2CH(CH_3)_2$ | H | H | CH | N | $n_D^{25}$ 1,4970 |

| Verb. Nr. | $R^1$ | $R^2$ | $R^3$ | $R^4$ | $R^5$ | X | Y | Phys. Konstante |
|---|---|---|---|---|---|---|---|---|
| 30 | $i-C_3H_7$ | H | $-C(CH_3)_2CH(CH_3)_2$ | H | H | N | N | $n_D^{25}$ 1,4860 |
| 31 | $i-C_3H_7$ | H | $-C(CH_3)_2CH(CH_3)_2$ | H | H | N | CH | $n_D^{25}$ 1,4910 |
| 32 | $i-C_3H_7$ | H | 1-Ethyl-1-cyclohexyl | H | H | CH | N | $n_D^{25}$ 1,5105 |
| 33 | $i-C_3H_7$ | H | 1-Ethyl-1-cyclohexyl | H | H | N | N | $n_D^{25}$ 1,5060 |
| 34 | $i-C_3H_7$ | H | 1-Ethyl-1-cyclohexyl | H | H | N | CH | $n_D^{25}$ 1,5105 |
| 35 | $CH_3-$ | $CH_3-$ | tert-Butyl | H | H | CH | N | Sdp. 110 - 112°C/ 0,2 mbar |
| 36 | $i-C_3H_7-$ | H | tert.-Butyl | H | H | CH | N | Sdp. 118 - 120°C/ 0,2 mbar |
| 37 | $C_2H_5-$ | $C_2H_5-$ | tert.-Butyl | H | H | CH | N | $n_D^{25}$ 1,5030 |
| 38 | $C_2H_5-$ | $C_2H_5-$ | tert.-Butyl | H | H | N | N | $n_D^{25}$ 1,4935 |
| 39 | $C_2H_5-$ | $C_2H_5-$ | tert.-Butyl | H | H | N | CH | $n_D^{25}$ 1,4930 |
| 40 | $-(CH_2)_5-$ | | tert.-Butyl | H | H | CH | N | Fp. 73 - 76°C |
| 41 | $-(CH_2)_5-$ | | tert.-Butyl | H | H | N | N | Fp. 80 - 82°C |
| 42 | $-(CH_2)_5-$ | | tert.-Butyl | H | H | N | CH | Fp. 41 - 43°C |
| 43 | $n-C_4H_9-$ | $C_2H_5$ | tert.-Butyl | H | H | CH | N | Sdp. 144 - 146°C/ 0,2 mbar |
| 44 | $n-C_4H_9-$ | $C_2H_5$ | tert.-Butyl | H | H | N | N | Sdp. 130 - 133°C/ 0,2 mbar |
| 45 | $n-C_4H_9-$ | $C_2H_5$ | tert.-Butyl | H | H | N | CH | Sdp. 124 - 126°C/ 0,2 mbar |

| Verb. Nr. | $R^1$ | $R^2$ | $R^3$ | $R^4$ | $R^5$ | X | Y | Phys. Konstante |
|---|---|---|---|---|---|---|---|---|
| 46 | $(CH_3)_3CCH_2CH(CH_3)-$ | H | tert.-Butyl | H | H | CH | N | Sdp. 148-151°C/0,3 mbar |
| 47 | $(CH_3)_3CCH_2CH(CH_3)-$ | H | tert.-Butyl | H | H | N | N | Sdp. 135-136°C/0,25 mbar |
| 48 | $i-C_3H_7$ | H | $-C(CH_3)_2C_3H_7-n$ | H | H | CH | N | $n_D^{25}$ 1,4915 |
| 49 | $i-C_3H_7$ | H | $-C(CH_3)_2C_3H_7-n$ | H | H | N | N | $n_D^{22}$ 1,4820 |
| 50 | $(CH_3)_3CCH_2CH(CH_3)-$ | H | $-C(CH_3)_2C_3H_7-n$ | H | H | CH | N | $n_D^{22}$ 1,4900 |
| 51 | $(CH_3)_3CCH_2CH(CH_3)-$ | H | $-C(CH_3)_2C_3H_7-n$ | H | H | N | N | $n_D^{22}$ 1,4825 |
| 52 | $n-C_{10}H_{23}$ | H | tert.-Butyl | H | H | CH | N | $n_D^{22}$ 1,4800 |
| 53 | $n-C_{10}H_{23}$ | H | tert.-Butyl | H | H | N | N | $n_D^{22}$ 1,4760 |
| 54 | $i-C_3H_7$ | H | n-Butyl | H | H | CH | N | Sdp. 148-152°C/0,4 mbar |
| 55 | $i-C_3H_7$ | H | n-Butyl | H | H | N | N | Sdp. 130-140°C/0,35 mbar |
| 56 | $i-C_3H_7$ | H | n-Hexyl | H | H | CH | N | $n_D^{22}$ 1,5029 |
| 57 | $i-C_3H_7$ | H | n-Hexyl | H | H | N | N | $n_D^{22}$ 1,4970 |

Die erfindungsgemäßen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Ferner können sie auch im Materialschutz, u. a. zur Bekämpfung holzzerstörender Pilze, wie Coniophora puteana und Polystictus versicolor, verwendet werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie Gemüse - wie Gurken, Bohnen und Kürbisgewächse -.

Die neuen Verbindungen sind insbesondere geeignet zur Bekämpfung folgender Pflanzenkrankheiten:
Pseudocercosporella herpotrichoides in Getreide, Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Rhynchosporium secalis und
Pyrenophora teres in Getreide
Alternaria solani an Tomaten
Pyricularia oryzae an Reis

Die Wirkstoffe können gleichzeitig das Wachstum von zwei oder mehr der genannten Pilze unterdrücken und besitzen eine hohe Pflanzenverträglichkeit. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen.

Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 bis 5 kg Wirkstoff je ha.

Die Wirkstoffe können auch zusammen mit anderen Wirkstoffen, z. B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d. h. die fungizide Wirksamkeit des Kombinationsproduktes ist größer als die der addierten Wirksamkeiten der Einzelkomponenten.

Fungizide, die mit den neuen Verbindungen kombiniert werden können, sind beispielsweise:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'propylen-bis-dithiocarbamat)
Zink-(N,N'-propylen-bis-dithiocarbamat)
N,N'-Polypropylen-bis-(thiocarbamoyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid

N-Trichlormethylthio-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-p-tert.-Butylphenyl)-2-methylpropyl)-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-propyl-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
-(2-Chlorphenyl)- -(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-glutaramid
Hexachlorbenzol
DL-Methyl-N-(2,6-dimethyl-phenyl)-N-furoyl(2)-alaninat
DL-N-(2,6-Dimethyl-phenyl)-N-(2'-methoxyacetyl)-alaninmethylester

N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid.
2-Cyano-N-(ethylaminocarbonyl)-2-methoximino)-acetamid
1-(2-(2,4-Dichlorphenyl)-pentyl-1H-1,2,4-triazol
2,4-Difluor-alpha-(1H-1,2,4-triazolyl-1-methyl)-benzhydrylalkohol

Die neuen Wirkstoffe werden beispielsweise in Form von direkt versprühbaren Lösungen, Suspensionen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, zum Beispiel Benzol, Toluol, Xylol, Paraffin, Tetrahydrnaphthalin, alkylierte Naphthaline oder deren Derivate z. B. Methanol, Ethanol, Propanol, Butanol, Chloroform, Tetrachlorkohlenstoff, Cyclohexanol, Cyclohexanon, Chlorbenzol, Isophoron usw., stark polare Lösungsmitte, wie z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl der Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze

der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, Octylphenol, Nonylphenol Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemitte, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

I. Man vermischt 90 Gewichtsteile der Verbindung 4 mit 100 Gewichtsteilen N-Methylpyrrolidon und erhält eine Lösung, die zur Anwendung in Form kleinster Tropfen geeignet ist.

II. 20 Gewichtsteile der Verbindung 18 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Doedecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung 21 werden in einer Mischung gelöst, die aus 30 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und Verrühren der Lösung in Wasser erhält man eine wäßrige Dispersion.

IV. 20 Gewichtsteile der Verbindung 22 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und Verrühren der Lösung in Wasser, erhält man eine wäßrige Dispersion.

V. 20 Gewichtsteile der Verbindung 25 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der Verbindung 29 werden mit 95 Gewichtsteilen feinteiligem Kaolin innig vermischt. Man erhält auf diese Weise ein Stäubemittel, das 5 Gew.-% des Wirkstoffs erhält.

VII. 30 Gewichtsteile der Verbindung 32 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung 36 werden mit 30 Teilen Natriumsalz eines Phenolsulfonsäure-Harnstoff-Formaldehyd-Kondensates, 2 Teilen Kieselgel und 48 Teilen Wasser innig vermischt. Man erhält eine stabile wäßrige Dispersion.

IX. 20 Teile der Verbindung 21 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd--Kondensates und 68 Teilen eines paraffinischen Mineralöls innig vermischt. Man erhält eine stabile ölige Dispersion.

Die folgenden Versuche belegen die biologische Wirkung der neuen Verbindungen. Vergleichsmittel A ist der bekannte, insbesondere zur Bekämpfung von Botrytis geeignete Wirkstoff N-Trichlormethylthio--tetrahydrophthalimid (Chem. Week, June 21, 1972, Seite 46).

Versuch 1

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise bei der Anwendung als 0,05 %ige Wirkstoffbrühe der Wirkstoff 21 eine bessere fungizide Wirkung zeigt (beispielsweise 90 %) als der bekannte Wirkstoff A (beispielsweise 70 %).

Versuch 2

Wirksamkeit gegen Gurkenmehltau

Blätter von in Töpfen gewachsenen Gurkenkeimlingen der Sorte "Chinesische Schlange" werden im Zweiblattstadium mit einer Sporensuspension des Gurkenmehltaus (Erysiphe cichoracearum) besprüht. Nach etwa 20 Stunden werden die Versuchspflanzen mit wäßriger Spritzbrühe, die 80 % Wirkstoff und 20 % Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 220°C und 70 bis 80 % relativer Luftfeuchtigkeit aufgestellt. Zur Beurteilung der Wirksamkeit der neuen Stoffe wird das Ausmaß der Pilzentwicklung nach 8 Tagen ermittelt.

Das Ergebnis des Versuches zeigt, daß beispielsweise bei der Anwendung als 0,025 %ige Wirkstoffbrühe die Wirkstoffe 4, 21, 22, 25, 29, 32 und 36 eine gute fungizide Wirkung zeigten (beispielsweise 100 %).

Versuch 3

Wirksamkeit gegen Pyricularia oryzae

Blätter von in Töpfen gewachsenen Reiskeimlingen der Sorte "Bahia" werden mit wäßrigen Emulsionen, die 80 % Wirkstoff und 20 % Emulgiermittel in der Trockensubstanz enthalten, tropfnaß besprüht und 24 Stunden später mit einer wäßrigen Sporensuspension von Pyricularia oryzae inoculiert.

Anschließend werden die Versuchspflanzen in Klimakammern bei 22 bis 24°C und 95 bis 99 % relativer Luftfeuchtigkeit aufgestellt. Nach 6 Tagen wird das Ausmaß des Krankheitsbefalls ermittelt.

Das Ergebnis des Versuches zeigt, daß beispielsweise bei der Anwendung als 0,05 %ige Wirkstoffbrühe der Wirkstoff 18 eine gute fungizide Wirkung zeigte (beispielsweise 90 %).

0171502

Patentansprüche

1. Vinylharnstoff der Formel

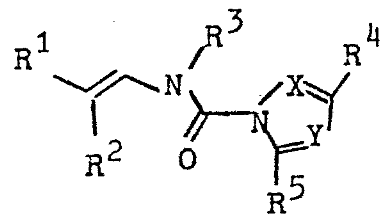

I,

in der $R^1$ und $R^2$ gleich oder verschieden sind und Wasserstoff oder einen gegebenenfalls durch Halogen oder Alkoxy substituierten Alkyl- oder Cycloalkylrest jeweils mit bis zu 12 Kohlenstoffatomen bedeuten oder $R^1$ zusammen mit $R^2$ Teil eines gegebenenfalls durch eine oder mehrere Alkylgruppen mit 1 bis 3 Kohlenstoffatomen substituierten 5- oder 6-gliedrigen carbocyclischen Ringes ist,
$R^3$ einen gegebenenfalls durch Halogen oder Alkoxy substituierten Alkyl- oder Cycloalkylrest jeweils mit bis zu 12 Kohlenstoffatomen,
X Stickstoff oder die Gruppe C-$R^6$,
Y Stickstoff oder CH, wobei mindestens einer der beiden Reste X und Y Stickstoff bedeutet,
$R^4$, $R^5$ und $R^6$ gleich oder verschieden sind und Wasserstoff, Halogen oder Alkyl mit bis zu 5 Kohlenstoffatomen bedeuten.

2. Fungizides Mittel, enthaltend Vinylharnstoff der Formel

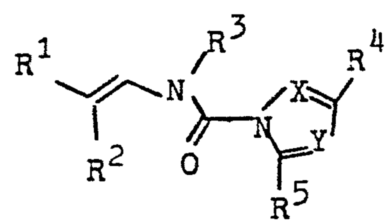

I,

in der $R^1$ und $R^2$ gleich oder verschieden sind und Wasserstoff oder einen gegebenenfalls durch Halogen oder Alkoxy substituierten Alkyl- oder Cycloalkylrest jeweils mit bis zu 12 Kohlenstoffatomen bedeuten oder $R^1$ zusammen mit $R^2$ Teil eines gegebenenfalls durch eine oder mehrere Alkylgruppen mit 1 bis 3 Kohlenstoffatomen substituierten 5- oder 6-gliedrigen carbocyclischen Ringes ist,
$R^3$ einen gegebenenfalls durch Halogen oder Alkoxy substituierten Alkyl- oder Cycloalkylrest jeweils mit bis zu 12 Kohlenstoffatomen,
X Stickstoff oder die Gruppe C-$R^6$,
Y Stickstoff oder CH, wobei mindestens einer der beiden Reste X und Y Stickstoff bedeutet,
$R^4$, $R^5$ und $R^6$ gleich oder verschieden sind und Wasserstoff, Halogen oder Alkyl mit bis zu 5 Kohlenstoffatomen bedeuten.

3. Fungizides Mittel, enthaltend einen inerten Zusatzstoff und Vinylharnstoff der Formel

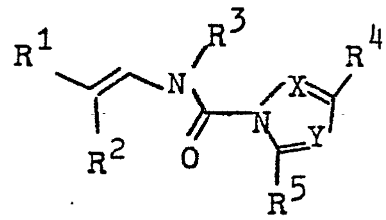

I,

in der $R^1$ und $R^2$ gleich oder verschieden sind und Wasserstoff oder einen gegebenenfalls durch Halogen oder Alkoxy substituierten Alkyl- oder Cycloalkylrest jeweils mit bis zu 12 Kohlenstoffatomen bedeuten oder $R^1$ zusammen mit $R^2$ Teil eines gegebenenfalls durch eine oder mehrere Alkylgruppen mit 1 bis 3 Kohlenstoffatomen substituierten 5- oder 6-gliedrigen carbocyclischen Ringes ist,
$R^3$ einen gegebenenfalls durch Halogen oder Alkoxy substituierten Alkyl- oder Cycloalkylrest jeweils mit bis zu 12 Kohlenstoffatomen,
X Stickstoff oder die Gruppe $C-R^6$,
Y Stickstoff oder CH, wobei mindestens einer der beiden Reste X und Y Stickstoff bedeutet,
$R^4$, $R^5$ und $R^6$ gleich oder verschieden sind und Wasserstoff, Halogen oder Alkyl mit bis zu 5 Kohlenstoffatomen bedeuten.

4. Verfahren zur Herstellung eines fungiziden Mittels, <u>dadurch gekennzeichnet</u>, daß man einen inerten Zusatzstoff vermischt mit Vinylharnstoff der Formel

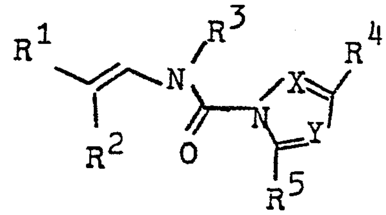

I,

in der $R^1$ und $R^2$ gleich oder verschieden sind und Wasserstoff oder einen gegebenenfalls durch Halogen oder Alkoxy substituierten Alkyl- oder Cycloalkylrest jeweils mit bis zu 12 Kohlenstoffatomen bedeuten oder $R^1$ zusammen mit $R^2$ Teil eines gegebenenfalls durch eine oder mehrere Alkylgruppen mit 1 bis 3 Kohlenstoffatomen substituierten 5- oder 6-gliedrigen carbocyclischen Ringes ist,
$R^3$ einen gegebenenfalls durch Halogen oder Alkoxy substituierten Alkyl- oder Cycloalkylrest jeweils mit bis zu 12 Kohlenstoffatomen,
X Stickstoff oder die Gruppe $C-R^6$,

Y Stickstoff oder CH, wobei mindestens einer der beiden Reste X und Y Stickstoff bedeutet,
$R^4$, $R^5$ und $R^6$ gleich oder verschieden sind und Wasserstoff, Halogen oder Alkyl mit bis zu 5 Kohlenstoffatomen bedeuten.

5. Verfahren zur Bekämpfung von Pilzen, dadurch gekennzeichnet, daß man die Pilze oder die von Pilzbefall bedrohten Materialien, Flächen, Pflanzen oder Saatgüter behandelt mit einer fungizid wirksamen Menge Vinylharnstoff der Formel

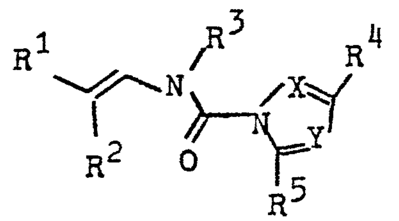 I,

in der $R^1$ und $R^2$ gleich oder verschieden sind und Wasserstoff oder einen gegebenenfalls durch Halogen oder Alkoxy substituierten Alkyl- oder Cycloalkylrest jeweils mit bis zu 12 Kohlenstoffatomen bedeuten oder $R^1$ zusammen mit $R^2$ Teil eines gegebenenfalls durch eine oder mehrere Alkylgruppen mit 1 bis 3 Kohlenstoffatomen substituierten 5- oder 6-gliedrigen carbocyclischen Ringes ist,
$R^3$ einen gegebenenfalls durch Halogen oder Alkoxy substituierten Alkyl- oder Cycloalkylrest jeweils mit bis zu 12 Kohlenstoffatomen,
X Stickstoff oder die Gruppe C-$R^6$,
Y Stickstoff oder CH, wobei mindestens einer der beiden Reste X und Y Stickstoff bedeutet,
$R^4$, $R^5$ und $R^6$ gleich oder verschieden sind und Wasserstoff, Halogen oder Alkyl mit bis zu 5 Kohlenstoffatomen bedeuten.

6. Verfahren zur Herstellung von Vinylharnstoff der Formel

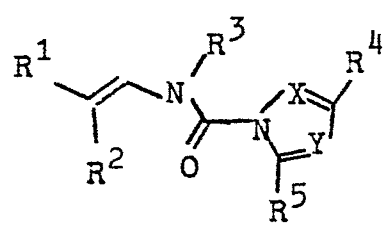 I,

in der $R^1$ und $R^2$ gleich oder verschieden sind und Wasserstoff oder einen gegebenenfalls durch Halogen oder Alkoxy substituierten Alkyl- oder Cycloalkylrest jeweils mit bis zu 12 Kohlenstoffatomen bedeuten oder $R^1$ zusammen mit $R^2$ Teil eines gegebenenfalls durch

eine oder mehrere Alkylgruppen mit 1 bis 3 Kohlenstoffatomen substituierten 5- oder 6-gliedrigen carbocyclischen Ringes ist,
$R^3$ einen gegebenenfalls durch Halogen oder Alkoxy substituierten Alkyl- oder Cycloalkylrest jeweils mit bis zu 12 Kohlenstoffatomen,
X Stickstoff oder die Gruppe $C-R^6$,
Y Stickstoff oder CH, wobei mindestens einer der beiden Reste X und Y Stickstoff bedeutet,
$R^4$, $R^5$ und $R^6$ gleich oder verschieden sind und Wasserstoff, Halogen oder Alkyl mit bis zu 5 Kohlenstoffatomen bedeuten, dadurch gekennzeichnet, daß man eine Verbindung der Formel

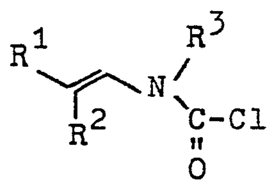
II,

in der $R^1$, $R^2$ und $R^3$ die im Anspruch 1 angegebenen Bedeutungen haben

a) mit Azolen der Formel

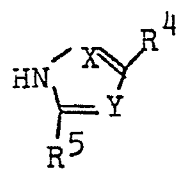
III,

in der $R^4$, $R^5$, X und Y die im Anspruch 1 angegebenen Bedeutungen haben, oder

b) mit deren Metall-Derivaten der Formel

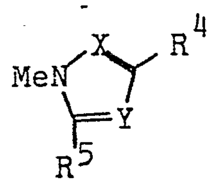
(IV),

in der $R^4$, $R^5$, X und Y die im Anspruch 1 angegebenen Bedeutungen haben und Me Lithium, Natrium, Kalium oder 1/2-Calcium bedeutet, oder

c) mit deren Silyl-Derivaten der Formel

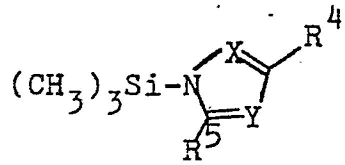 (V),

in der $R^4$, $R^5$, X und Y die im Anspruch 1 angegebenen Bedeutungen haben, umsetzt.

7. Vinylharnstoff der Formel I gemäß Anspruch 1, dadurch gekennzeichnet, daß $R^1$ und $R^2$ Wasserstoff, Methyl, Ethyl, Propyl, Isopropyl, Butyl, sek.-Butyl, iso-Butyl, tert.-Butyl, n-Pentyl, 2-Methylbutyl, 2,2-Dimethylpropyl, n-Hexyl, 2,2,3-Trimethylpropyl, 3,3-Dimethylbutyl, n-Heptyl, n-Octyl, 2,4,4-Trimethylpentyl, 2,2,3,3-Tetramethylbutyl, 2-Ethylhexyl, n-Nonyl, 3,5,5-Trimethylhexyl, 2-Isopropyl-5-methylhexyl, n-Decyl, 3,7-Dimethyloctyl oder Dodecyl bedeuten oder
$R^1$ zusammen mit $R^2$ Teil eines gegebenenfalls durch Methyl, Ethyl, Propyl Isopropyl, n-Butyl, tert.-Butyl substituierten Cyclopentyl- oder Cyclohexylringes ist,
$R^3$ tert.-Butyl, 2-Methylbutyl-2, 2-Ethylpentyl-2, 1,1,2-Trimethylpropyl-1, 1,1-Dimethylbutyl-1, 1,1-Dimethylpentyl-1, 1,1,2,2-Tetramethylpropyl-1, 1-Methyl-1-cyclopentyl, 1-Ethyl-1-cyclopentyl, 1--Propyl-1-cyclopentyl, 1-Methyl-1-cylohexyl, 1-Ethyl-1-cyclohexyl, 1,4-Dimethyl-1-cyclohexyl, 1-Methyl-4-tert.-butyl-1-cyclohexyl, 1--Ethyl-4-methyl-1-cyclohexyl,

$R^4$, $R^5$ und $R^6$ Wasserstoff, Chlor, Brom, Methyl, Ethyl, n-Propyl oder Isopropyl, X Stickstoff oder die Gruppe C-$R^6$ und Y Stickstoff oder CH bedeutet.

Patentansprüche

1. Fungizides Mittel, enthaltend - Vinylharnstoff der Formel

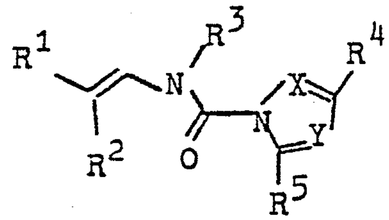 I,

in der $R^1$ und $R^2$ gleich oder verschieden sind und Wasserstoff oder einen gegebenenfalls durch Halogen oder Alkoxy substituierten Alkyl- oder Cycloalkylrest jeweils mit bis zu 12 Kohlenstoffatomen bedeuten oder $R^1$ zusammen mit $R^2$ Teil eines gegebenenfalls durch eine oder mehrere Alkylgruppen mit 1 bis 3 Kohlenstoffatomen substituierten 5- oder 6-gliedrigen carbocyclischen Ringes ist,
$R^3$ einen gegebenenfalls durch Halogen oder Alkoxy substituierten Alkyl- oder Cycloalkylrest jeweils mit bis zu 12 Kohlenstoffatomen,
X Stickstoff oder die Gruppe C-$R^6$,
Y Stickstoff oder CH, wobei mindestens einer der beiden Reste X und Y Stickstoff bedeutet,
$R^4$, $R^5$ und $R^6$ gleich oder verschieden sind und Wasserstoff, Halogen oder Alkyl mit bis zu 5 Kohlenstoffatomen bedeuten.

2. Fungizides Mittel, enthaltend einen inerten Zusatzstoff und Vinylharnstoff der Formel

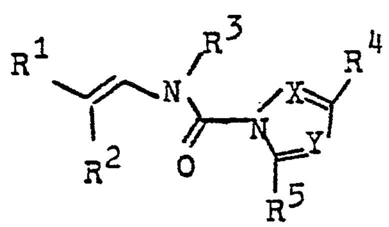 I,

in der $R^1$ und $R^2$ gleich oder verschieden sind und Wasserstoff oder einen gegebenenfalls durch Halogen oder Alkoxy substituierten Alkyl- oder Cycloalkylrest jeweils mit bis zu 12 Kohlenstoffatomen bedeuten oder $R^1$ zusammen mit $R^2$ Teil eines gegebenenfalls durch eine oder mehrere Alkylgruppen mit 1 bis 3 Kohlenstoffatomen substituierten 5- oder 6-gliedrigen carbocyclischen Ringes ist,
$R^3$ einen gegebenenfalls durch Halogen oder Alkoxy substituierten Alkyl- oder Cycloalkylrest jeweils mit bis zu 12 Kohlenstoffatomen,
X Stickstoff oder die Gruppe C-$R^6$,

Y Stickstoff oder CH, wobei mindestens einer der beiden Reste X und Y Stickstoff bedeutet,
$R^4$, $R^5$ und $R^6$ gleich oder verschieden sind und Wasserstoff, Halogen oder Alkyl mit bis zu 5 Kohlenstoffatomen bedeuten.

3. Verfahren zur Herstellung eines fungiziden Mittels, dadurch gekennzeichnet, daß man einen inerten Zusatzstoff vermischt mit Vinylharnstoff der Formel

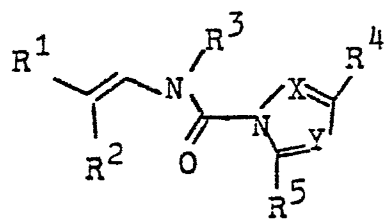
I,

in der $R^1$ und $R^2$ gleich oder verschieden sind und Wasserstoff oder einen gegebenenfalls durch Halogen oder Alkoxy substituierten Alkyl- oder Cycloalkylrest jeweils mit bis zu 12 Kohlenstoffatomen bedeuten oder $R^1$ zusammen mit $R^2$ Teil eines gegebenenfalls durch eine oder mehrere Alkylgruppen mit 1 bis 3 Kohlenstoffatomen substituierten 5- oder 6-gliedrigen carbocyclischen Ringes ist,
$R^3$ einen gegebenenfalls durch Halogen oder Alkoxy substituierten Alkyl- oder Cycloalkylrest jeweils mit bis zu 12 Kohlenstoffatomen,
X Stickstoff oder die Gruppe C-$R^6$,
Y Stickstoff oder CH, wobei mindestens einer der beiden Reste X und Y Stickstoff bedeutet,
$R^4$, $R^5$ und $R^6$ gleich oder verschieden sind und Wasserstoff, Halogen oder Alkyl mit bis zu 5 Kohlenstoffatomen bedeuten.

4. Verfahren zur Bekämpfung von Pilzen, dadurch gekennzeichnet, daß man die Pilze oder die von Pilzbefall bedrohten Materialien, Flächen, Pflanzen oder Saatgüter behandelt mit einer fungizid wirksamen Menge Vinylharnstoff der Formel

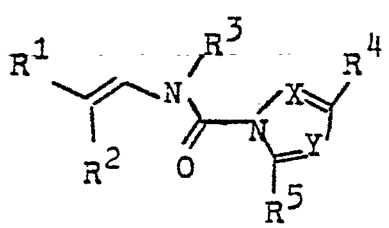
I,

in der $R^1$ und $R^2$ gleich oder verschieden sind und Wasserstoff oder einen gegebenenfalls durch Halogen oder Alkoxy substituierten Alkyl- oder Cycloalkylrest jeweils mit bis zu 12 Kohlenstoffatomen bedeuten oder $R^1$ zusammen mit $R^2$ Teil eines gegebenenfalls durch

eine oder mehrere Alkylgruppen mit 1 bis 3 Kohlenstoffatomen substituierten 5- oder 6-gliedrigen carbocyclischen Ringes ist,
$R^3$ einen gegebenenfalls durch Halogen oder Alkoxy-substituierten Alkyl- oder Cycloalkylrest jeweils mit bis zu 12 Kohlenstoffatomen,
X Stickstoff oder die Gruppe $C-R^6$,
Y Stickstoff oder CH, wobei mindestens einer der beiden Reste X und Y Stickstoff bedeutet,
$R^4$, $R^5$ und $R^6$ gleich oder verschieden sind und Wasserstoff, Halogen oder Alkyl mit bis zu 5 Kohlenstoffatomen bedeuten.

5. Verfahren zur Herstellung von Vinylharnstoff der Formel

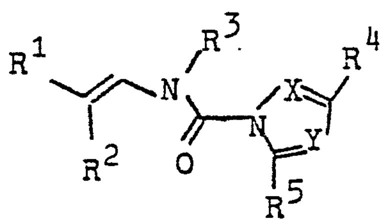

I,

in der $R^1$ und $R^2$ gleich oder verschieden sind und Wasserstoff oder einen gegebenenfalls durch Halogen oder Alkoxy substituierten Alkyl- oder Cycloalkylrest jeweils mit bis zu 12 Kohlenstoffatomen bedeuten oder $R^1$ zusammen mit $R^2$ Teil eines gegebenenfalls durch eine oder mehrere Alkylgruppen mit 1 bis 3 Kohlenstoffatomen substituierten 5- oder 6-gliedrigen carbocyclischen Ringes ist,
$R^3$ einen gegebenenfalls durch Halogen oder Alkoxy substituierten Alkyl- oder Cycloalkylrest jeweils mit bis zu 12 Kohlenstoffatomen,
X Stickstoff oder die Gruppe $C-R^6$,
Y Stickstoff oder CH, wobei mindestens einer der beiden Reste X und Y Stickstoff bedeutet,
$R^4$, $R^5$ und $R^6$ gleich oder verschieden sind und Wasserstoff, Halogen oder Alkyl mit bis zu 5 Kohlenstoffatomen bedeuten, dadurch gekennzeichnet, daß man eine Verbindung der Formel

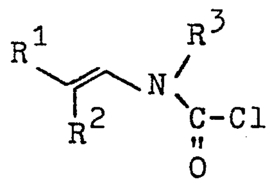

II,

in der $R^1$, $R^2$ und $R^3$ die im Anspruch 1 angegebenen Bedeutungen haben

a) mit Azolen der Formel

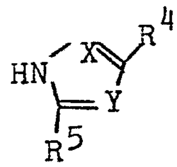

III,

in der $R^4$, $R^5$, X und Y die im Anspruch 1 angegebenen Bedeutungen haben, oder

b) mit deren Metall-Derivaten der Formel

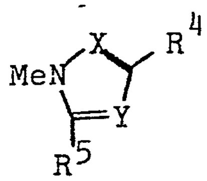

(IV),

in der $R^4$, $R^5$, X und Y die im Anspruch 1 angegebenen Bedeutungen haben und Me Lithium, Natrium, Kalium oder 1/2-Calcium bedeutet, oder

c) mit deren Silyl-Derivaten der Formel

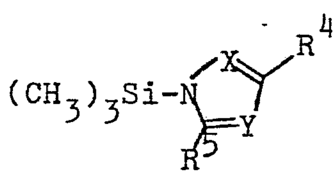

(V),

in der $R^4$, $R^5$, X und Y die im Anspruch 1 angegebenen Bedeutungen haben, umsetzt.